Europäisches Patentamt

European Patent Office

Office européen des brevets

(19)

(11) Numéro de publication: **0 167 984**
**B1**

(12) **FASCICULE DE BREVET EUROPEEN**

(45) Date de publication du fascicule du brevet:
30.03.88

(21) Numéro de dépôt: **85108263.6**

(22) Date de dépôt: **04.07.85**

(51) Int. Cl.⁴: **C 12 P 5/02, C 12 M 1/00**

(54) **Procédé pour obtenir du méthane par fermentation d'algues.**

(30) Priorité: **09.07.84 FR 8410869**

(43) Date de publication de la demande:
**15.01.86 Bulletin 86/3**

(45) Mention de la délivrance du brevet:
**30.03.88 Bulletin 88/13**

(84) Etats contractants désignés:
**BE CH DE FR GB IT LI NL SE**

(56) Documents cités:
**FR - A - 2 516 542**
**US - A - 4 022 665**

**BIOMASSE ACTUALITE, no. 12A, mai 1983, pages 36-37, Langres, FR; A. BORIES et al.:"Digestion anaérobie de biomasses végétales aquatiques"**

(73) Titulaire: **COMPAGNIE GENERALE D'ELECTRICITE Société anonyme dite:, 54, rue La Boétle, F-75382 Paris Cedex 08 (FR)**

(72) Inventeur: **Petitbon, Alain, 23 rue des Menuets, F-78730 St Arnoult en Yvelines (FR)**

(74) Mandataire: **Weinmiller, Jürgen et al, Lennéstrasse 9 Postfach 24, D-8133 Feldafing (DE)**

# Description

La présente invention concerne un procédé pour obtenir du méthane par fermentation d'algues.

Les algues vertes prolifèrent aussi bien dans les eaux douces que salées et constituent souvent une source de pollution par engorgement des cours d'eaux ou dépôt indésirable sur les plages côtières. De telles algues peuvent être soumises à une fermentation anaérobie dans une cuve fermée maintenue à une température de l'ordre de 37°C pendant 20 jours environ. Cette fermentation fournit un gaz riche en méthane qu'on peut recueillir à travers une soupape ménagée sur la paroi de la cuve. Par ce procédé connu on obtient ainsi au maximum 100 litres de méthane par kilogramme d'algues sèches. Comme les dépenses énergétiques nécessitées par l'entretien de la fermentation représentent 50 à 60% de l'énergie calorifique qu'il est possible de récupérer par combustion du méthane, un tel procédé n'est pas économiquement rentable.

Un autre procédé connu, tel qu'il est décrit par exemple dans le document FR-A-2 516 542, vise la fermentation d'un mélange d'algues et d'autres matières organiques telles que de la tourbe ou des ordures ménagères.

Dans le document US-A-4 022 665, on décrit également un procédé de traitement d'ordures et de biomasse. La fermentation a lieu dans une ambiance acide.

La présente invention a pour but d'augmenter le rendement énergétique des procédés de ce type tout en diminuant le temps de fermentation de façon à rendre la récupération du méthane.

Ce but est atteint par le procédé tel qu'il est défini par la revendication 1. En ce qui concerne des caractéristiques d'une mise en œuvre préférée de l'invention, référence est faite aux sous-revendications.

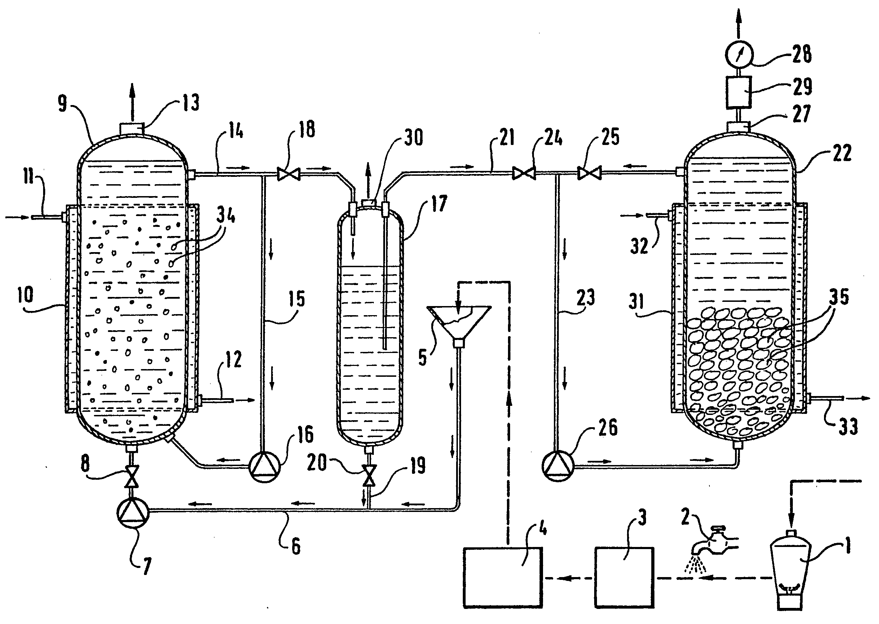

Des modes particuliers de mise en œuvre du procédé selon l'invention sont décrits ci-dessous, à titre d'exemple, en regard du dessin annexé dans lequel la figure unique représente schématiquement un dispositif capable de mettre en œuvre le procédé selon l'invention.

Sur cette figure est représenté un poste de hachage 1 suivi successivement d'un poste de lavage 2, d'un poste de traitement préalable physico-chimique 3 qui sera précisé ultérieurement et d'un poste de préchauffage 4.

A la sortie du poste 4 est disposé un récipient conique 5 dont le goulot est relié à une canalisation 6 sur laquelle sont disposées en série successivement une pompe 7 et une vanne 8. L'extrémité de la canalisation 6 sortant de la vanne 8 débouche au fond d'une cuve 9 de forme cylindrique à axe vertical. Sur la surface cylindrique de la cuve 9 est fixée une enveloppe annulaire 10 qui peut contenir un liquide circulant entre une entrée 11 et une sortie 12. L'extrémité supérieure de la cuve 9 est fermée par une paroi munie d'une soupape 13. De la partie supérieure de la cuve 9 part une canalisation 14 sur laquelle est branchée en dérivation une autre canalisation 15 qui débouche dans le fond de la cuve 9. Une pompe péristaltique 16 est disposée en série sur la canalisation 15.

La canalisation 14 provenant de la cuve 9 débouche à la partie supérieure d'une autre cuve fermée 17, de forme cylindrique à axe vertical. Une vanne 18 est placée en série sur la canalisation 14 entre sa jonction avec la canalisation 15 et la cuve 17. L'extrémité supérieure de la cuve 17 est munie d'une soupape 30. Le fond de la cuve 17 est relié par une canalisation 19 à la canalisation 6, en un point situé entre le récipient 5 et la pompe 7. Une vanne 20 est placée en série sur la canalisation 19.

Une canalisation 21 dont une extrémité est située à l'intérieur de la cuve 17, sensiblement à mi-hauteur de cette cuve, traverse la paroi supérieure de la cuve 17, l'autre extrémité de la canalisation 21 débouchant à la partie supérieure d'une autre cuve 22 de forme cylindrique à axe vertical. Une canalisation 23 est branchée en dérivation sur la canalisation 21 en un point situé dans sa partie extérieure à la cuve 17 et débouche à la partie inférieure de la cuve 22. Deux vannes 24 et 25 sont placées en série sur la canalisation 21, la vanne 24 étant située entre la cuve 17 et la jonction des canalisations 21 et 23, et la vanne 25 étant située entre cette jonction et la cuve 22. Une pompe 26 doseuse à membrane est placée en série sur la canalisation 23.

La paroi supérieure de la cuve 22 est munie d'une soupape 27 dont la sortie est reliée à un compteur 28 à travers un système d'épuration 29. Sur la surface cylindrique de la cuve 22 est fixée une enveloppe annulaire 31 munie d'une entrée 32 et d'une sortie 33 de liquide.

Le dispositif décrit ci-dessus et illustré par la figure fonctionne de la manière suivante.

Des algues vertes, ramassées par exemple sur une plage au bord de la mer, sont amenées d'abord au poste de hachage 1 pour les diviser en fragments dont la dimension est de l'ordre du millimètre. Les algues hachées sont ensuite soumises à un lavage à l'eau au poste 2, de façon à éliminer le sable qu'elles peuvent comporter. Après ce lavage, les algues sont soumises à un traitement physico-chimique dans le poste 3. Le traitement a pour effet de diviser les fragments d'algues en très fines particules de façon à augmenter la surface spécifique de la phase solide de ces algues. La dimension des particules est suffisamment faible pour dépolymériser partiellement la matière solide des algues; certaines chaines unissant les molécules du matériau polymère des algues se trouvent ainsi rompues. Le traitement effectué dans le poste 3 peut comporter un broyage très fin et (ou) un traitement dans un bain de soude suivie d'une neutralisation à l'acide. Après ce traitement physico-chimique, la suspension ainsi obtenue est de préférence préchauffée dans le poste 4 à une température de l'ordre de 37°C.

La suspension sortant du poste 4 est versée dans le récipient conique 5, la vanne 20 étant fermée, la vanne 8 ouverte et la pompe 7 étant mise en marche, de façon à introduire la suspension dans la cuve 9. Cette cuve contient des granulés d'alumine poreuse 34, sur lesquels sont fixés des enzymes qui peuvent être constitués par un mélange de cellulases et d'hémicellulases dont les activités sont adaptées à la transformation en sucres du matériau organique des algues. Les granulés peuvent par exemple être constitués d'alumine gamma, avoir une dimension comprise entre 100 et 200 micromètres et présenter une surface spécifique de 100 m² par gramme. La fixation des enzymes sur les granulés peut être effectuée préalablement par agitation des granulés dans une solution enzymatique tamponnée à pH 4,5.

L'introduction de la suspension dans la cuve 9 contenant les granulés crée un lit fluidisé qui est maintenu à une température de 37°C par circulation d'un fluide caloporteur dans l'enveloppe 10 de son entrée 11 à sa sortie 12.

De préférence la suspension contenue dans le lit fluidisé est soumise à un recyclage permanent qui s'effectue par circulation à travers les canalisations 14 et 15 dans le sens indiqué par les flèches, après mise en marche de la pompe 16, la vanne 18 étant fermée.

De plus un appareil relié à un capteur (non représentés) plongé dans la cuve 9 mesure le pH de la suspension, celui-ci étant maintenu à une valeur sensiblement égale à 6.

Le passage de la suspension dans le lit fluidisé transforme en sucres la majeure partie de la phase organique de la suspension. Comme le liquide de la suspension comporte des bactéries d'acidogénèse provenant des algues introduites dans le dispositif, ces bactéries transforment les sucres en acides gras volatils. Bien entendu, le taux d'enzymes immobilisés dans les granulés est choisi de sorte que la concentration en sucres dans la suspension n'atteigne pas le taux d'inhibition de ces enzymes.

La soupape 13 permet d'évacuer le gaz carbonique qui se dégage au cours de ces réactions.

Le séjour de la suspension dans le lit fluidisé dure quelques jours, cinq jours au maximum.

La suspension ayant subi le traitement d'acidogénèse dans la cuve 9 est alors transférée dans la cuve de décantation 17 à travers la canalisation 14, après ouverture de la vanne 18 et arrêt de la pompe 16. Au cours de la décantation, les particules non transformées en acide se concentrent progressivement dans le fond de la cuve 17. La soupape 30 permet d'évacuer le gaz carbonique qui se dégage. La décantation est arrêtée au bout d'un intervalle de temps compris entre 10 et 20 heures.

La partie supérieure de la cuve 17 contient alors un liquide décanté constitué par une solution d'acides gras volatils qui est aspirée en dehors de la cuve à travers la canalisation 21, après ouverture de la vanne 24, fermeture de la vanne 25 et mise en marche de la pompe 26.

Les résidus de décantation concentrés dans la partie inférieure de la cuve 17 peuvent être renvoyés dans la cuve 9 après ouverture des vannes 20 et 8 et mise en marche de la pompe 7.

La solution d'acides gras aspirée à travers la canalisation 21 circule à travers la canalisation 23 dans le sens de la flèche et se trouve injectée à la base de la cuve 22. Cette cuve contient un lit fixe bactérien constitué, par exemple, par un empilement d'anneaux 35 en céramique poreuse, dans lesquels sont immobilisés des bactéries méthanogènes. Ces bactéries peuvent provenir de celles présentes dans les algues qui alimentent le poste de hachage 1, après culture dans un milieu approprié. Elles sont immobilisées dans le lit fixe par adsorption sur des sites actifs superficiels des anneaux. Ceux-ci peuvent être en alumine alpha frittée à 1400°C et présentent des pores de 10 micromètres environ.

De préférence on effectue un recyclage de la solution d'acides gras qui traverse le lit fixe, en fermant la vanne 24 et en ouvrant la vanne 25, la pompe 26 étant maintenue en marche.

Pendant toute la durée du passage du liquide décanté à travers le lit fixe, la température de la solution est maintenue à une valeur de 37°C par circulation d'un liquide caloporteur dans l'enveloppe 31 de son entrée 32 à sa sortie 33. De même le pH de la solution est maintenu à une valeur sensiblement égale à 7,5.

Le passage du liquide décanté dans la cuve 22 à travers le lit fixe provoque le dégagement d'un gaz composé de plus de 70% de méthane, de gaz carbonique, de vapeur d'eau et éventuellement d'un peu de H₂S si les algues contiennent du soufre. Ce gaz sort de la cuve par la soupape 27 et traverse un système d'épuration 29 dans lequel la vapeur d'eau est absorbée par un produit approprié et H₂S est fixé sur du charbon actif. Un gaz contenant donc principalement du méthane et un peu de gaz carbonique traverse le compteur 28 pour être utilisé comme combustible.

Le temps de passage du liquide décanté dans la cuve 22 peut être réduit à 5 jours environ. Le taux de demande chimique en oxygène (D.C.O.) peut être réduit de 90%, 95% des acides gras volatils étant consommés.

La durée totale du séjour des algues dans le dispositif représenté sur la figure est donc de 10 jours environ; elle est donc deux fois moins longue que la durée totale de fermentation dans le procédé connu cité plus haut. De plus le procédé selon l'invention permet d'obtenir environ 200 litres de méthane par kilogramme d'algues sèches, soit deux fois plus qu'avec le procédé connu. Dans ces conditions, la récupération d'énergie par combustion du méthane devient rentable.

Ces résultats peuvent s'expliquer par les considérations suivantes.

Dans le procédé selon l'invention, le traitement préalable qui réduit les algues en une suspension de fines particules, de structure cristalline simplifiée, entraîne une augmentation considérable de la vitesse des réactions enzymatiques et bactériennes. Les réactions d'acidogénèse et de méthanogénèse sont réalisées dans deux cuves distinctes maintenues à des taux d'acidité différentes (pH 6 et 7,5): ces réactions s'effectuent ainsi avec un rendement optimal. Enfin les apports d'enzymes dans le lit fluidisé et de bactéries dans le lit fixe permettent de diminuer de façon importante le temps de séjour des algues dans le dispositif.

A titre indicatif, le procédé selon l'invention peut être appliqué au traitement d'ulves récoltées par exemple sur des plages du département des Côtes-du-Nord (France).

Les ulves égouttées sont hachées pendant une minute dans un mixeur, puis broyées pendant cinq minutes dans un broyeur à billes. Les billes du broyeur sont de préférence en céramique pour éviter une pollution toxique pour les bactéries. Le broyage peut être remplacé par un traitement à froid, pendant 10 à 15 heures, par une solution de soude à 3 ou 4%, ce traitement étant suivi d'une neutralisation à l'acide acétique jusqu'à l'obtention du pH égal à 6, requis pour l'acidogénèse. Le coût de la consommation de soude et d'acide acétique est partiellement compensé par une augmentation du rendement, car l'acétate formé est transformé en méthane.

La suspension introduite dans le bas de la cuve d'acidogénèse par la pompe péristaltique comprend 10% environ de matière sèche. L'hydrolyse enzymatique est effectuée avec un taux de 2 à 3% d'enzymes actives et 30% de granulés d'alumine, en poids, par rapport à la matière sèche. Dans les granulés est fixé un mélange enzymatique comprenant principalement des cellulases et des hémicellulases à activité glucosidase, xylosidase et rhamnosidase. Il est à noter que les enzymes fixées conservent leur pleine activité pendant au moins quinze jours, puis certaines activités décroissent lentement. La durée du séjour dans la cuve d'acidogénèse est de cinq jour durant lesquels une proportion de 63% de la matière organique est solubilisée contre 43% en 6 jours dans une acidogénèse effectuée suivant le procédé connu, sans traitement préalable.

Après 15 heures de décantation, la solution d'acides gras volatils, qui comprend essentiellement de l'acide acétique, de l'acide propionique et de l'acide butyrique est injectée dans le bas de la cuve de méthanogénèse. Après 5 jours de temps de rétention, on obtient un volume de méthane égal au volume de la cuve de méthanogénèse, c'est-à-dire environ 250 litres de méthane par kilogramme de matière sèche. Le taux de D.C.O. est réduit de plus de 90% et les acides gras sont consommés dans une proportion supérieure à 95%.

Le gaz récupéré contient 75% de méthane.

Le procédé selon l'invention peut être aussi appliqué à la fermentation d'entéromorphes cultivées sur eaux résiduaires.

**Revendications**

1. Procédé pour obtenir du méthane par fermentation d'algues, caractérisé en ce qu'il comporte successivement les étapes suivantes:
— traitement préalable des algues, capable de les transformer en une suspension de fines particules solides dans un liquide, ce traitement étant capable en outre de dépolymériser partiellement la matière solide des algues,
— passage de la suspension dans un lit fluidisé contenant des granulés (34) sur lesquels sont immobilisés des enzymes aptes à transformer les particules en sucres, le liquide contenant des bactéries acidogènes aptes à trasnformer ces sucres en acides gras volatils,
— décantation de la suspension, de manière à éliminer les particules solides restantes et à prélever un liquide décanté,
— et passage du liquide décanté à travers un lit fixe contenant des bactéries méthanogènes fixées sur un support, de manière à provoquer dans ce liquide un dégagement d'un mélange gazeux contenant principalement du méthane.

2. Procédé selon la revendication 1, caractérisé en ce que le traitement préalable comporte un broyage.

3. Procédé selon la revendication 1, caractérisé en ce que le traitement préalable comporte un traitement à la soude.

4. Procédé selon la revendication 1, caractérisé en ce que le passage de la suspension dans le lit fluidisé comporte un recyclage.

5. Procédé selon la revendication 1, caractérisé en ce que la décantation de la suspension comporte un recyclage vers le lit fluidisé des résidus de décantation.

6. Procédé selon la revendication 1, caractérisé en ce que le passage du liquide décanté à travers le lit fixe comporte un recyclage.

7. Procédé selon la revendication 1, caractérisé en ce que le taux d'enzymes immobilisés dans les granulés (34) est choisi de sorte que la concentration en sucres dans le liquide n'atteigne pas le seuil d'inhibition des enzymes.

8. Procédé selon la revendication 1, caractérisé en ce que la température de la suspension dans le lit fluidisé et celle du liquide décanté dans le lit fixe sont maintenues à une valeur constante de 37°C.

9. Procédé selon la revendication 1, caractérisé en ce que le pH de la suspension dans le lit fluidisé est maintenu à 6.

10. Procédé selon la revendication 1, caractérisé en ce que le pH du liquide décanté passant à travers le lit fixe est maintenu à 7,5.

11. Procédé selon la revendication 1, caractérisé en ce que les granulés (34) sont en alumine gamma poreuse.

12. Procédé selon la revendication 1, caractérisé en ce que le support des bactéries méthanogènes est constitué par des anneaux (35) de céramique poreuse.

13. Procédé selon la revendication 12, caractérisé en ce que la céramique poreuse est constituée par de l'alumine alpha frittée.

**Patentansprüche**

1. Verfahren zur Gewinnung von Methan durch Gärung von Algen, gekennzeichnet durch die folgenden Schritte:
— Vorbehandlung der Algen derart, dass sie in eine Suspension feiner Festkörperartikel in einer Flüssigkeit umgewandelt werden, wobei die Behandlung weiter zur partiellen Depolymerisation der festen Materie der Algen geeignet ist,
— Durchleitung der Suspension durch ein Fliessbett von Granulat (34), auf welchem Enzyme zur Umwandlung der Partikel in Zucker immobilisiert sind, wobei die Flüssigkeit säurebildende Bakterien zur Umwandlung des Zuckers in flüchtige Fettsäuren enthält,
— Dekantierung der Suspension derart, dass die festen Restpartikel ausgeschieden und eine dekantierte Flüssigkeit entnommen wird, und

— Durchleitung der dekantierten Flüssigkeit durch ein Festbett mit auf Trägern fixierten methanogenen Bakterien, derart, dass in der Flüssigkeit die Abscheidung eines Gasgemisches ausgelöst wird, welches hauptsächlich Methan enthält.

2. Verfahren nach Anspruch 1, dadurch gekennzeichnet, dass die Vorbehandlung mit einem Mahlgang verbunden ist.

3. Verfahren nach Anspruch 1, dadurch gekennzeichnet, dass die Vorbehandlung eine Behandlung mit Soda einschliesst.

4. Verfahren nach Anspruch 1, dadurch gekennzeichnet, dass die Durchleitung der Suspension durch das Fliessbett eine Rückführung einschliesst.

5. Verfahren nach Anspruch 1, dadurch gekennzeichnet, dass die Dekantierung der Suspension mit einer Rückführung der Reststoffe der Dekantierung in das Fliessbett verbunden ist.

6. Verfahren nach Anspruch 1, dadurch gekennzeichnet, dass die Durchleitung der dekantierten Flüssigkeit durch das Festbett mit einer Rückführung verbunden ist.

7. Verfahren nach Anspruch 1, dadurch gekennzeichnet, dass der Anteil der immobilisierten Enzyme im Granulat (34) so gewählt ist, dass die Zuckerkonzentration in der Flüssigkeit nicht die Inhibitionsschwelle der Enzyme erreicht.

8. Verfahren nach Anspruch 1, dadurch gekennzeichnet, dass die Temperatur der Suspension im Fliessbett und der dekantierten Flüssigkeit im Festbett auf einem konstanten Wert von 37°C gehalten wird.

9. Verfahren nach Anspruch 1, dadurch gekennzeichnet, dass der pH-Wert der Suspension im Fliessbett bei 6 gehalten wird.

10. Verfahren nach Anspruch 1, dadurch gekennzeichnet, dass der pH-Wert der durch das Festbett geleiteten dekantierten Flüssigkeit bei 7,5 gehalten wird.

11. Verfahren nach Anspruch 1, dadurch gekennzeichnet, dass das Granulat (34) aus poröser Gammatonerde besteht.

12. Verfahren nach Anspruch 1, dadurch gekennzeichnet, dass der Träger der methanogenen Bakterien aus porösen Keramikringen (35) besteht.

13. Verfahren nach Anspruch 12, dadurch gekennzeichnet, dass die poröse Keramik aus gesinterter Alphatonerde besteht.

## Claims

1. A process for obtaining methane by fermenting algae, characterized in that it involves the following successive stages:

— pre-treatment of the algae, capable of producing a liquid suspension of fine solid particles, said treatment being moreover capable of partially depolymerizing the solid algae matter,

— running the suspension through a fluidized bed containing granules (34) on which enzymes are immobilized which are capable of transforming the particles into sugar, said liquid containing acidific bacteria capable of transforming said sugars into volatile fatty acids,

— decantation of the suspension, so as to remove any solid particles that may remain, and to extract a decanted liquid, and

— running the decanted liquid across a fixed bed containing methanogenic bacteria set onto a support so as to cause the liquid to release a gas mixture containing mainly methane.

2. A process according to claim 1, characterized in that the pre-treatment involves a milling operation.

3. A process according to claim 1, characterized in that the pre-treatment involves a soda treatment.

4. A process according to claim 1, characterized in that running the suspension through the fluidized bed involves recycling operations.

5. A process according to claim 1, characterized in that decanting the suspension involves recycling the residue from decantation to the fluidized bed.

6. A process according to claim 1, characterized in that running the decanted liquid across the fixed bed involves recycling operations.

7. A process according to claim 1, characterized in that the amount of enzymes immobilized in the granules (34) is selected so that the sugar concentration in the liquid does not reach the threshold beyond which the enzymes are inhibited.

8. A process according to claim 1, characterized in that the fluidized bed suspension and the decanted fixed bed liquid are kept at a constant temperature of 37°C.

9. A process according to claim 1, characterized in that the pH level of the fluidized bed suspension is kept at 6.

10. A process according to claim 1, characterized in that the pH level of the decanted fixed bed liquid is kept at 7.5.

11. A process according to claim 1, characterized in that the granules (34) are made of porous gamma alumina.

12. A process according to claim 1, characterized in that the methanogenic bacteria support is made up of porous ceramic rings (35).

13. A process according to claim 12, characterized in that said porous ceramic material is sintered alpha alumina.

0 167 984